(19) Europäisches Patentamt / European Patent Office / Office européen des brevets

(11) **EP 4 288 007 B1**

(12) **EUROPÄISCHE PATENTSCHRIFT**

(45) Veröffentlichungstag und Bekanntmachung des Hinweises auf die Patenterteilung:
**18.12.2024 Patentblatt 2024/51**

(21) Anmeldenummer: **22709604.7**

(22) Anmeldetag: **04.02.2022**

(51) Internationale Patentklassifikation (IPC):
***A61F 9/008*** (2006.01)

(52) Gemeinsame Patentklassifikation (CPC):
**A61F 9/008; A61F 9/00821;** A61B 2018/00803;
A61F 2009/00844; A61F 2009/00863

(86) Internationale Anmeldenummer:
**PCT/EP2022/052669**

(87) Internationale Veröffentlichungsnummer:
**WO 2022/167562 (11.08.2022 Gazette 2022/32)**

(54) **LASERTHERAPIEVORRICHTUNG ZUR THERAPIE EINES LEBENDEN GEWEBES**

LASER THERAPY DEVICE FOR THERAPY OF A LIVING TISSUE

DISPOSITIF DE THÉRAPIE LASER DESTINÉ À LA THÉRAPIE D'UN TISSU VIVANT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.02.2021 DE 102021201080**

(43) Veröffentlichungstag der Anmeldung:
**13.12.2023 Patentblatt 2023/50**

(73) Patentinhaber: **Medizinisches Laserzentrum Lübeck GmbH**
**23562 Lübeck (DE)**

(72) Erfinder:
- **SEIFERT, Eric**
  **23554 Lübeck (DE)**
- **BRINKMANN, Ralf**
  **23562 Lübeck (DE)**
- **ABBAS, Hossameldin**
  **23562 Lübeck (DE)**

(74) Vertreter: **RCD-Patent**
**Giesen, Schmelcher & Griebel**
**Patentwanwälte PartG mbB**
**Postfach 3106**
**52118 Herzogenrath (DE)**

(56) Entgegenhaltungen:
**US-A1- 2006 111 697    US-A1- 2013 102 894**

- **HEIKE H. MÜLLER ET AL: "Imaging thermal expansion and retinal tissue changes during photocoagulation by high speed OCT", BIOMEDICAL OPTICS EXPRESS, vol. 3, no. 5, 1 May 2012 (2012-05-01), United States, pages 1025, XP055692336, ISSN: 2156-7085, DOI: 10.1364/BOE.3.001025**

## Beschreibung

Die Erfindung betrifft eine Vorrichtung zur Lasertherapie eines lebenden Gewebes aufweisend eine gepulst betriebene Laserlichtquelle und eine automatische Regelung der eingestrahlten Leistung. Die Erfindung betrifft insbesondere eine ophthalmologische Laservorrichtung zur Therapie des Augenhintergrundes.

Laser werden zur Behandlung verschiedenster Netzhauterkrankungen eingesetzt. Bei der Laserbestrahlung des Augenhintergrundes werden primär die lichtabsorbierenden Schichten, das retinale Pigmentepithel (RPE) und die Aderhaut (Choroid), erwärmt, von wo aus sich die Wärme in die angrenzenden Schichten ausbreitet. Die Ausbreitung hängt dabei neben der absorbierten Laserenergie von der Pulsdauer und Fläche der Bestrahlung ab. Pro Laserspot an der Netzhaut liegt die typische Fläche bei 100-500 $\mu$m im Durchmesser bei einer Bestrahlungszeit von meistens 20-500 ms. Je kürzer die Bestrahlungszeit, desto weniger Wärme diffundiert in das Volumen außerhalb der bestrahlten Region.

Je nach therapeutischer Zielrichtung sind unterschiedliche Temperaturen wünschenswert, um eine Unterbehandlung, d.h. das Ausbleiben eines therapeutischen Effektes, oder eine Überbehandlung, d.h. unbeabsichtigte Kollateralschäden, zu vermeiden. Im genannten Zeitbereich werden zur subletalen Stimulierung der Netzhaut ca. 43-50 °C benötigt (z.B. hyperthermische Gewebestimulierung zur prophylaktischen Therapie trockener altersbedingter Makuladegeneration AMD), zur subsichtbaren (subthreshold) 50-70 °C oder milden Photokoagulation 70-90 °C (z.B. diabetische Makulopathie), für starke Photokoagulation (panretinale Bestrahlung zur diabetischen Retinopathie oder Netzhautablösungen) 90-120 °C. Zur selektiven thermomechanischen Disruption des RPEs (z.B. für Retinopathia Centralis Serosa) werden Nano- bis Mikrosekundenpulse eingesetzt, wobei die Temperaturen > 140 °C nur im RPE auftreten (selektive Retinatherapie, SRT) und dort zur selektiven Erzeugung von Mikroblasen an den intrazellulären Melanosomen führen. Ab 2 $\mu$s Pulsdauer wurde neben der Disruption auch thermische Nekrose beobachtet. Generell gelten Pulsdauern zwischen 2 und 50 $\mu$s als Übergangsbereich von thermomechanischen zu thermischen Effekten.

Aufgrund der starken Schwankungen der Lichttransmission durch das Auge und die sehr variable Lichtabsorption des RPEs (20-80 % im sichtbaren Spektralbereich) sind die oben genannten Temperaturerhöhungen nicht mit festen Lasereinstellungen erzielbar. Daher sind die Messung und die Regelung der Temperaturerhöhung während der Bestrahlung unbedingt wünschenswert.

Zur Temperaturmessung ist beispielsweise aus der Druckschrift US 2003/032949 A1 ein optoakustisches Verfahren bekannt, bei dem mittels repetitiv kurz gepulster Laserstrahlung (Pulsdauer im Nanosekundenbereich, Repetitionsrate typisch 1 kHz), die der Therapie-laserstrahlung überlagert wird, die Netzhautabsorber thermoelastisch angeregt werden, und die emittierten Drucktransienten an der Hornhaut des Auges mittels eines Ultraschallwandlers abgegriffen werden können. Aufgrund der Temperaturabhängigkeit des Grüneisen-Koeffizienten steigen die Druckamplituden mit der Netzhauttemperatur an und geben, nach vorhergehender Kalibrierung, die aktuelle mittlere Temperaturerhöhung an der Netzhaut an. Streng genommen ist eine Temperaturbestimmung auf diese Weise nur möglich, solange das Zielgewebe lediglich reversibel erwärmt, aber noch nicht thermisch umgewandelt und dauerhaft geschädigt wird. Gleichwohl sind Konzepte entwickelt worden, um Gewebeschädigungen durch eine Laserpulssequenz ("Burst") einzuschätzen und insbesondere dabei Temperaturbestimmungen während einer ersten Teilsequenz des Bursts vorzunehmen, um den Laser während des Applizierens einer zweiten Teilsequenz desselben Bursts zu regeln, vgl. beispielsweise Druckschrift US 2010/292763 A1.

Die Zielvorstellung ist, dass der behandelnde Arzt den Therapielaser für jeden zu behandelnden Laserspot nur genau einmal auslösen muss und dabei davon ausgehen kann, dass eine beabsichtigte Gewebeschädigung ebendort eintritt ohne übermäßige Kollateralschäden zu erzeugen. In der Regel entstehen durch diese "schonende" Lasertherapie nur schwach oder gar nicht sichtbare Läsionen auf der Netzhaut. Dem Arzt steht insofern auch keine unmittelbare und objektive Effektkontrolle seiner Behandlung zur Verfügung.

Ein offenkundiger Nachteil des Verfahrens der US 2003/032949 A1 ist die Notwendigkeit zur Nutzung eine separaten Messlasers neben dem Therapielaser, was Komplexität und Kosten des Lasersystems erhöht.

Derzeit sind kontinuierlich emittierende high power Laser mit Emissionsleistungen der Größenordnung 10 W im grünen Spektralbereich für Anwendungen an der Netzhaut in Entwicklung. Im vorliegenden Kontext sind Emissionsleistungen der Größenordnung von 5 W an Emissionsleistungen von der Erfindung eingeschlossen. Es wird erwartet, dass in Zukunft auch Laser mit Emissionsleistungen der Größenordnung 100 W und annähernd konstanter Leistung über längere Zeitdauer für medizinische Zwecke verfügbar werden. Bei Anwendung derart hoher Leistungen muss die Gesamtbestrahlungszeit pro Laserspot im unteren $\mu$s-Zeitbereich (2-50 $\mu$s) oder sogar noch darunter liegen und sehr gut geregelt werden, um keine starken, unkontrollierbaren Schäden an der Netzhaut zu erzeugen. Insbesondere kann bei der Nutzung solcher Laser anstelle einer Laserpulssequenz bereits ein Einzelpuls therapiewirksam sein.

Das sehr schnelle Aufheizen des Gewebes macht es unmöglich, ein optoakustisches Verfahren nach Art der US 2003/032949 A1 zur Temperaturkontrolle zu verwenden, weil die Drucktransienten grundsätzlich bis zu ca. 20 $\mu$s Laufzeit von der Retina zu einem Schallaufnehmer in einem Kontaktglas benötigen. Das Messsignal käme zu spät an, um den Heizlaserpuls noch zu regeln.

Man könnte alternativ auf eine optische Detektion der transienten Volumenänderung des bestrahlten Gewebes durch reversible thermische Expansion bzw. Kontraktion unter Erwärmung mit Laserlicht setzen. Solche Verfahren wurden erprobt, beispielsweise in den Druckschriften US 2013/102894 A1 und US 2015/011983 A1, und nutzen Interferometer und eigens zur Interferometrie vorgesehene, üblich in den Strahlengang des Therapielasers eingespiegelte Messlichtquellen. Auch hier liegen die Nachteile aber in erster Linie in der Komplexität und in den Kosten des Lasersystems.

Aus der Arbeit von Xiaohua Feng et al., "Selftemperature regulation of photothermal therapy by laser-shared photoacoustic feedback", Vol. 40, No. 19, October 1st 2015, Optics Letters, S. 4492-4495, geht eine Laservorrichtung mit photoakustischem (= optoakustischem) Feedback zur Temperaturkontrolle des Targets mit nur einem Heizlaser hervor. Dieser Laser wird pulsmoduliert betrieben, wobei sich lange Heizpulse und linear gechirpte Sequenzen kurzer Pulse zur Temperaturmessung via Drucktransienten mit einem Ultraschallwandler zeitlich fortlaufend abwechseln. Es gelingt dadurch ein selbsttätiges Einregeln der Bestrahlungsdosis auf das Target durch Modulation der Pulsdauern des Heizlasers, so dass das Target vorbestimmte Temperaturen mit hoher Präzision erreicht und beibehält. In dieser Arbeit ist das Target ein Phantom, die Emissionsleistung des Lasers beträgt 1 W und die Heizlaserpulse weisen Pulsdauern im Millisekunden- bis Sekundenbereich auf. Die Chirp-Sequenz nimmt 40 Mikrosekunden in Anspruch, und es tritt keine Laufzeitverzögerung zum Ultraschallwandler auf. Diese vereinfachten Bedingungen lassen keine unmittelbare Anwendbarkeit auf das Problem der Retinatherapie zu.

Die Idee, das Heizlaserlicht selbst auch zur Temperaturbestimmung heranzuziehen, wird hier wie schon in der US 2010/292763 A1 verfolgt. Die Druckschrift DE 10 2009 016184 A1 beschreibt einen Ansatz mit abwechselnd applizierten kurzen Messlaserpulsen und sehr viel länger dauernden Heizlaserpulsen aus einer gemeinsamen Laserquelle konkret zur Lasertherapie des Augenhintergrunds unter Temperaturüberwachung und -regelung.

Die Arbeit zur photoakustischen Bildgebung von Fei Gao et al., "Single laser pulse generates dual photoacoustic signals for differential contrast photoacoustic imaging", Scientific Reports | 7: 626 | DOI:10.1038/s41598-017-00725-4, online erschienen am 04.04.2017, offenbart die Möglichkeit der photoakustischen Bildgebung eines biologischen Gewebes anhand von nur zwei Drucktransienten, die am Anfang und am Ende eines langen Heizlaserpulses ausgelöst und detektiert werden. Unter Verzicht auf Druckeinschluss ("stress confinement") aber unter Beibehaltung von Wärmeeinschluss ("thermal confinement") zeigt sich, dass die Drucktransienten von Anfang und Ende des Heizlaserpulses entgegengesetzte Polarität und unterschiedliche Amplituden aufweisen, die Aufschluss über die während der Pulsdauer im Gewebe deponierten Energiedichte geben können.

Vor diesem Hintergrund stellt sich die Erfindung die erste Aufgabe, eine Lasertherapievorrichtung zur Therapie eines lebenden Gewebes vorzuschlagen, die eine selbstgeregelte, schonende Therapie ausschließlich mit Heizlaserpulsen, d.h. mit Laserpulsen von relativ hoher Leistung und Pulsdauer, ermöglicht. Als zweite Aufgabe der Erfindung soll die Vorrichtung dazu fortgebildet werden, eine objektive Effekteinschätzung zu ermöglichen.

Die erste Aufgabe wird gelöst durch eine Lasertherapievorrichtung zur Therapie eines lebenden Gewebes aufweisend eine gepulst betriebene Laserlichtquelle emittierend Laserlicht mit einer Emissionsleistung aus dem Intervall von 5 W bis 100 W, eine Einrichtung zur Führung des Laserlichts in einen Applikator, eine Auslöseeinrichtung zum Auslösen der Gewebebestrahlung mit dem Applikator, eine Detektionseinrichtung zur Messung der durch Laserabsorption im Gewebe ausgelösten zeitabhängigen Volumenänderungen, eine Recheneinheit zur Bewertung der detektierten Volumenänderungen und zur Ausgabe von Steuerbefehlen an eine Steuereinrichtung zur Steuerung der in das Gewebe eingestrahlten Leistung des Laserlichts, dadurch gekennzeichnet, dass

a. jedes Auslösen der Gewebebestrahlung die Steuereinrichtung veranlasst, einen ersten Heizlaserpuls mit einer ersten Leistung und einer ersten Pulsdauer und wenigstens einen zweiten Heizlaserpuls mit einer zweiten Leistung und einer zweiten Pulsdauer in einem vorbestimmten zeitlichen Pulsabstand auf das Gewebe zu applizieren, wobei

b. die Detektionseinrichtung die durch die ansteigende und die abfallende Leistungsflanke des ersten Heizlaserpulses bedingten Volumenänderungen optisch oder akustisch erfasst und die Messwerte der Recheneinheit zuführt;

c. die Recheneinheit basierend auf den Messwerten der Volumenänderung unter Berücksichtigung wenigstens der vorbestimmten Steigungen der Leistungsflanken des ersten Heizlaserpulses einen Schätzwert für die Temperaturerhöhung des Gewebes während der Einstrahlung des ersten Heizlaserpulses ermittelt und

d. die Recheneinheit aus dem Schätzwert einen Befehl an die Steuereinrichtung erzeugt, der die Steuereinrichtung veranlasst, die zweite Leistung und/oder die zweite Pulsdauer des zweiten Heizlaserpulses so einzurichten, dass die Einstrahlung des zweiten Heizlaserpulses das Gewebe bis auf eine vorbestimmte Zieltemperatur erwärmt.

Die Unteransprüche sind auf vorteilhafte Ausgestaltungen gerichtet, insbesondere auch auf Fortbildungen zur Effekteinschätzung.

Bisher wurden zur Temperaturbestimmung üblich kurze, energiearme Laserpulse verwendet, die für sich genommen nicht oder nur wenig zur Erwärmung des Gewebes beitragen sollten. Es war generell beabsichtigt, die Zustandsänderung des Gewebes bei seiner Zustandsbestimmung möglichst zu vermeiden.

Die erfindungsgemäße Vorrichtung verzichtet nun darauf, kurze Laserpulse oder Bursts solcher Laserpulse zu applizieren, sondern appliziert vielmehr ausschließlich lange Heizlaserpulse mit mittlerer bis hoher Laserleistung, von denen jeder einzelne eine Temperaturerhöhung um etliche Grad Celsius über seine Pulsdauer hinweg nach sich zieht.

Zur Behandlung eines einzelnen Laserspots - insbesondere auf der Retina - braucht es dann nur sehr wenige Pulse, vorzugsweise sogar nur gerade mal zwei.

Der erste Heizlaserpuls, der einen Laserspot erwärmt, soll vorzugsweise noch keine dauerhafte Gewebeschädigung (z.B. Denaturierung) auslösen, sondern vielmehr nur ausreichend schnelle Volumenänderungen durch thermische Expansion des Gewebes am Anfang und Kontraktion am Ende des Heizlaserpulses hervorrufen, die mit einer optischen oder akustischen Detektionseinrichtung erfasst werden können. Die Detektionseinrichtungen erzeugen dabei elektrische Volumenänderungssignale, die an eine Recheneinheit übermittelt und von dieser ausgewertet werden können.

Die Detektionseinrichtung kann beispielsweise zur optischen Erfassung von thermisch im bestrahlten Gewebe ausgelösten Bewegungen von Licht streuenden oder reflektierenden Gewebeschichten ausgebildet sein und ein Interferometer sowie wenigstens einen Photodetektor umfassen, der eine zeitlich veränderliche Lichtintensität misst und als Messwerte ausgibt. Hierfür können zum Beispiel optische Kohärenztomographie (OCT) eingesetzt werden oder auch eine Lichtquelle hoher Kohärenz (single longitudinal mode) mit Speckle-Erfassung. Der Therapielaser selbst kann auch Licht mit ausreichend hoher Kohärenz emittieren, so dass ein kleiner Anteil des Therapielichts zugleich Messlicht sein kann. Aus den ermittelten Bewegungen - etwa der Änderung des Abstandes zweier streuender Gewebeschichten - lässt sich direkt auf die gesuchte Volumenänderung schließen.

Es ist erfindungsgemäß bevorzugt, dass die Detektionseinrichtung zur Erfassung von thermisch im bestrahlten Gewebe ausgelösten Druckwellen ausgebildet ist und wenigstens einen Ultraschallwandler umfasst, der eine Drucktransiente erfasst und als Messwerte ausgibt. Den Ultraschallwellen steht sowohl während der langen Pulse als auch zwischen den Pulsen ausreichend Zeit zur Propagation vom erwärmten Gewebe zu den Ultraschallaufnehmern zur Verfügung.

Es ist erfindungsgemäß vorgesehen, den zweiten Heizlaserpuls, der erst nach der Erfassung und Auswertung der Volumenänderungssignale appliziert wird, mit den Informationen aus dem ersten Heizlaserpuls zu regeln.

Bevorzugt liegen die erste und zweite Pulsdauer im Intervall von 2 bis 50 Mikrosekunden. Andere Pulsdauern, insbesondere kürzere, sind aber nicht ausgeschlossen. Die Unterscheidbarkeit - Trennbarkeit - der Volumenänderungssignale von Pulsanfang und Pulsende ist abhängig von der Art der Detektion; optische Detektion lässt dies auch für sehr kurze Pulse zu. Die akustische Detektion ist apparativ zumeist simpler.

Der Pulsabstand ist üblich definiert als der Zeitabstand gleichartiger Features zweier aufeinander folgender Laserpulse, also beispielsweise jeweils der ansteigenden Pulsflanken oder der Pulsmaxima. Bei wenigen Pulsen mit jeweils großen Pulsdauern ist es hingegen zweckmäßig, den Pulsabstand als die Dauer der Pause zwischen zwei Pulsen zu definieren, also den Zeitabstand zwischen der abfallenden Flanke eines Heizlaserpulses und der ansteigenden Flanke des nächsten Heizlaserpulses. Vorzugsweise soll der zeitliche Pulsabstand zwischen dem ersten und dem wenigstens zweiten Heizlaserpuls größer als die thermische Relaxationszeit des Gewebes vorbestimmt sein.

Für die thermische Relaxationszeit bestehen Vorerkenntnisse in der Literatur, z.B. Brinkmann und Birngruber, "Selektive Retina-Therapie", in Z. Med. Phys 17 (2007), S. 6-22. Dort ist zu entnehmen, dass die thermische Relaxationszeit von Größe und Struktur des mit Laserlicht geheizten Objekts abhängt und insbesondere für eine angenommen homogene RPE-Absorberschicht von $4\,\mu m$ Dicke etwa $64\,\mu s$ beträgt. Die Autoren schlussfolgern weiter: "Ist es Ziel, die gesamten RPE-Zellen gleichmäßig zu erhitzen, ohne die Umgebung nennenswert zu erwärmen, so bieten sich Pulsdauern von ca. $5\text{-}30\,\mu s$ an. Will man hingegen ausgeprägte Spitzentemperaturen an den Melanosomen erzeugen, sollten Pulsdauern $< 5\,\mu s$ gewählt werden. Bei Pulsdauern $> 50\,\mu s$ geht die zellulär begrenzte Temperaturerhöhung verloren." Hieraus ergibt sich auch das oben genannte, bevorzugte Intervall für die Pulsdauern.

Besonders bevorzugt ist der zeitliche Pulsabstand so groß gewählt, dass die mit dem ersten Heizlaserpuls erreichte Temperaturerhöhung des Gewebes vor Auslösen des zweiten Pulses bereits durch Wärmedissipation weitgehend aufgehoben worden ist, d.h. dass das Gewebe auf die Basistemperatur des Gewebes vor Beginn der Bestrahlung, typisch 37 °C, zurückgekehrt ist.

Während der erste Heizlaserpuls der Informationsgewinnung über das Gewebe im gewählten Laserspot dient, bewirkt erfindungsgemäß der wenigstens zweite Heizlaserpuls die Erwärmung ebendort auf eine Zieltemperatur für einen therapeutischen Effekt. Der zweite Heizlaserpuls ist üblich energiereicher als der erste Heizlaserpuls, d.h. das Produkt aus Laserleistung und Pulsdauer ist für den zweiten Heizlaserpuls typisch größer als für den ersten Heizlaserpuls.

Es wird als vorteilhaft betrachtet, dass der erste und der zweite Heizlaserpuls dieselbe Pulsdauer aufweisen und für den zweiten Heizlaserpuls allein die Laserleistung geregelt wird. Dies ist bei den bislang entwickelten Laserdioden mit hohen Leistungen ohne Weiteres durch die Regelung der direkten Bestromung zu erreichen. Dadurch werden teure optische Komponenten zur elektronisch gesteuerten Abschattung oder Ablenkung des Laserlichts, wie z.B. ein akusto-optischer Modulator, unnötig. Gleichwohl liegt die Verwendung von Lichtmodulatoren zur Variation der applizierten Leistung ebenfalls im Rahmen der vorliegenden Erfindung.

Unter der Voraussetzung, dass die Emissionsleistung der Laserlichtquelle beim Ein- und Ausschalten eines Heizlaserpulses je eine ausreichend steile ansteigende und absteigende Flanke und dazwischen einen glatten, möglichst konstanten Plateauverlauf aufweist, werden tatsächlich nur zwei signifikante Volumenänderungssignale erzeugt, sofern die während der Flankendauer, $\delta t$, eingestrahlte Energie mit den typischen Pulsenergien von Probepulsen der bisherigen Detektion korrespondiert. Für die photoakustische Detektion sind beispielsweise Probepulse der Energie 5 $\mu$J als ausreichend bekannt. Bei einer Laserleistung von $P_{max}$ = 10 W entspricht dies einer Flankendauer von $\delta t$ = 1 $\mu$s, in der die applizierte Laserleistung von null auf den Maximalwert in erster Näherung linear hochfährt bzw. wieder abfällt.

Als ganz besonderer Vorzug der Erfindung wird angesehen, dass die Auswertung der Volumenänderung infolge des ersten Heizlaserpulses zur Ermittlung eines Schätzwertes für die Temperaturerhöhung ohne weiteres ganz genauso auch auf den wenigstens zweiten Heizlaserpuls angewendet werden kann, so dass sich nach dem Ende der Bearbeitung eines Laserspots ein Schätzwert für die tatsächlich therapeutisch wirksam durch den Laser hervorgerufene Zieltemperatur ergibt. Dieser Schätzwert kann dem behandelnden Arzt in Echtzeit angezeigt und überdies protokolliert, d.h. elektronisch gespeichert, und mit der vom Arzt gewünschten Zieltemperatur verglichen werden. Der Nutzer hat somit eine unmittelbare Effektkontrolle und auf Wunsch ein gespeichertes Therapieprotokoll zur Verfügung, das er auch als elektronischen Behandlungsnachweis oder zur Dokumentation einer Therapiehistorie verwenden kann.

Appliziert man Heizlaserpulse im $\mu$s Zeitregime mit Laserleistungen im W-Bereich auf absorbierendes Gewebe, so ergeben sich während der Leistungsänderungen dP1 und dP2 des Lasers - an der aufsteigenden (dP1) und absteigenden (dP2) Laserpulsflanke - dann gut detektierbare, schnelle Volumenänderungen durch Expansion dV1 und Kontraktion dV2 des Gewebes, wenn die Leistungsänderungen schnell genug erfolgen innerhalb kurzer Flankendauern dt1 und dt2, respektive. Die Heizlaserpulsflanken bedingen so die zu erfassenden Messsignale, nämlich als die Gewebeantwort unmittelbar auf das An- und Abschalten des Lasers. Bei Pulsen mit konstantem Plateauverlauf genügt die Berücksichtigung der Steigungen der Laserleistung an den Pulsflanken zur Signalauswertung.

Die Geschwindigkeiten der Expansion dV1/dt1 und Kontraktion dV2/dt2 sind optischinterferometrisch direkt messbar, indem man Positionsverschiebungen von Gewebeschichten, beispielsweise der Gewebeoberfläche, verfolgt. Während der Flanken des Heizlaserpulses sind diese Geschwindigkeiten am größten und gestatten die Einschätzung des Wärmeausdehnungskoeffizienten des Gewebes, der seinerseits als temperaturabhängige Materialgröße über einen breiten Temperaturbereich aus Vorarbeiten entnommen werden kann.

Zugleich lösen die schnellen Volumenänderungen Druckwellen im bestrahlten Gewebe aus, die durch das Gewebe propagieren und als zeitabhängiges Drucksignal (Drucktransiente) von einem Ultraschallaufnehmer registriert werden können. In diesem Fall nutzt man die ebenfalls vorbekannte Temperaturabhängigkeit des Grüneisen-Koeffizienten, um unmittelbar aus den Drucktransienten auf die Temperatur zu schließen. Als konkrete Messgrößen dD1 und dD2 kommen z.B. die maximale Amplitude der Drucktransiente oder das Integral der Drucktransiente über einen Zeitbereich erhöhter Amplitude oder auch die Frequenzen der Drucktransienten in Betracht.

Es wurde experimentell mit akustischer Detektion gefunden, dass das Verhältnis der Druckmesssignale von Laserpulsende zu Laserpulsanfang als ein Maß für die Erwärmung des Gewebes während des Heizlaserpulses benutzt werden kann, wenn die Leistungsänderungen und Flankendauern an beiden Pulsenden dem Betrage nach gleich sind. Dies ist aber in der Regel nicht der Fall. Man kann jedoch die Laserpulsflanken entweder bei jedem Puls messen oder - bei ausreichender Stabilität des Lasers von Puls zu Puls - aus vorab tabellierten Daten entnehmen, die bereits der Hersteller der Laserlichtquelle bereitstellen könnte.

Bei unterschiedlichen Flankenverläufen am Anfang und am Ende des Heizlaserpulses kann das Verhältnis der Druckmesssignale auf die Flankensteigungen normiert werden, beispielsweise gemäß:

$$\alpha = \frac{dD2/_{\Delta P2}}{dD1/_{\Delta P1}}$$

Die Größe $\Delta P$ kann hier als die maximale Steigung der Laserflanken $\Delta P = \frac{dP}{dt}(t = t_{max})$ oder alternativ als die mittlere Steigung

$$\Delta P = \frac{1}{\delta t}\int_0^{\delta t}\frac{dP}{dt}(t')\,dt'$$

gemittelt über die Flankendauer $\delta t$ eingeführt werden. Auch andere Normierungen in diesem Sinne sind möglich.

Je steiler die Leistungsänderungen des Laserlichts sind, desto schneller läuft die Volumenänderung des Gewebes ab. Dies bewirkt dann höhere Expansions- und Kontraktionsgeschwindigkeiten und höhere Druckamplituden.

In Bezug auf die akustische Detektion ist der Wert ermittelbar, der, in anderen Worten ausgedrückt, die Verhältnisse der Energien der ausgelösten Druckwellen zu den eingestrahlten Laserenergien jeweils für Pulsendflanke und Pulsanfangsflanke zueinander ins Verhältnis setzt. Aus dem Verhältnis a und der bekannten Temperaturabhängigkeit des Grüneisen-Koeffizienten des Gewebes kann die erzeugte Temperaturerhöhung des Gewebes bis zum Pulsende abgeleitet werden.

Insbesondere bei Pulsdauern, die kleiner als die thermische Relaxationszeit des Gewebes sind, ergibt sich ein Temperaturgradient in Strahlausbreitungsrichtung z gemäß der Absorption und Streuung der Strahlung $\mu a(z)$ und $\mu s(z)$. Der Wert $\alpha$ führt daher nur auf einen mittleren gewichteten Temperaturwert, der von allen Bestrahlungs- und Gewebeparametern abhängt.

Um näherungsweise auf die Temperaturerhöhung zu schließen muss der temperaturabhängige Verlauf des Grüneisen-Koeffizienten sowie die Starttemperatur vor Bestrahlung (Körpertemperatur, Raumtemperatur) bekannt sein. Die Temperaturabhängigkeit des Grüneisen-Koeffizienten kann z.B. mit einer quadratischen Gleichung der Form

$$\Gamma(T) = C * [(T^2 - T_0^2) - 2T_m(T - T_0)]$$

für Gewebe im Temperaturbereich von 0 - 100 °C angenähert werden. Für den Fall von Netzhautgewebe von Schweinen in einer Küvette wurde das Maximum der Parabel $T_m$ = 105.4°C, und der Nulldurchgang $T_0$ = -21°C approximiert (aus: Kandulla et al, Journal of Biomedical Optics 11(4) 2006), wobei C eine Proportionalitätskonstante ist.

Eine Möglichkeit der Berechnung der mittleren Temperaturerhöhung im Gewebe, $\overline{T_{Helz}}$, besteht darin, diese über eine Fit-Funktion direkt zu berechnen. Die mittlere Temperaturerhöhung ergibt sich aus der o.g. parabolischen Näherung zu

$$\overline{T_{Helz}} = T_m \pm \sqrt{\alpha B + (T_m - T_0)^2}$$

mit

$$B = T_B^2 - T_0^2 - 2T_m \cdot (T_B - T_0)$$

Bei $T_B$ handelt es sich um die Basistemperatur (z.B. Raumtemperatur oder Körpertemperatur). Um aus der mittleren Temperaturerhöhung $\overline{T_{Helz}}$ auf die oft interessantere maximale Temperaturerhöhung $T_{max}$ am Ort der höchsten Temperatur schließen zu können kann eine Konstante f bestimmt werden.

$$T_{max} = (\overline{T_{Helz}} - T_B) \cdot f + T_B$$

Die Konstante f hängt im Allgemeinen von der Bestrahlungsgeometrie und Bestrahlungsdauer ab, sowie von den thermischen Gewebeparametern. Im Fall nur eines ausgedehnten homogenen Absorbers ohne Streuung bei Bestrahlung mit Pulsdauern unterhalb der thermischen Relaxationszeit ergibt sich f = 2. Allgemein kann f für spezielle Probentypen bzw. hier Gewebeklassen über theoretische Modelle oder auch experimentell (z.B. mittels Thermofühler, temperaturabhängig fluoreszierende Nanopartikel etc.) ermittelt werden.

Zur Steuerung des Bestrahlungsprozesses auf eine definierte Zieltemperatur, $T_{ziel}$, werden mindestens zwei Heizlaserpulse appliziert, wobei der erste Heizlaserpuls eine Energie $E_1$ hat, der die Zieltemperatur noch nicht erreichen lässt und vorzugsweise auch keine thermische Schädigung des Gewebes bewirkt. Aus der nach dem ersten Puls gemessenen Temperatur $\overline{T_{Heiz}}$ kann die Energie $E_2$ des zweiten Heizlaserpulses über Einrichten der Leistung und/oder der Pulsdauer so angepasst werden, dass die Zieltemperatur nach Ende des zweiten Pulses erreicht wird, beispielsweise durch

$$T_{Ziel} = (\overline{T_{Heiz}} - T_B) * \frac{E_2}{E_1} + T_B$$

Es können auch mehrere Pulse zur stufenweisen Annäherung an die Zieltemperatur appliziert werden.

Solange die durch Laserabsorption im Gewebe deponierte Wärmeenergie das bestrahlte Volumen nicht via Wärmetransportmechanismen verlassen kann (thermischer Einschluss), spielt es im Prinzip keine Rolle, ob die erhöhte Energie $E_2$ durch Steigerung der Leistung oder der Pulsdauer des wenigstens einen zweiten Heizlaserpulses erzielt wird. Relevant sind zunächst nur die Produkte aus Leistung und Pulsdauer. Allerdings wird es als vorteilhaft angesehen, die zweite Pulsdauer nicht zu erhöhen, sondern vielmehr gleich der ersten Pulsdauer zu setzen und allein die Leistung zu steigern. Der Wärmetransport ist eben zeitabhängig, und die Transportprozesse während des zweiten Heizlaserpulses sind denen des ersten Heizlaserpulses bei gleicher Pulsdauer naturgemäß am ähnlichsten. Zudem zeigt sich bei der Stromregelung der Laserlichtquelle, das Ein- und Ausschaltvorgänge zu unterschiedlichen Laserleistungsverläufen bei verschiedenen Pulsdauern führen können. Wenn man auf einen Lichtmodulator zur Steuerung der Emission des kontinuierlich aktivierten Lasers verzichten will und stattdessen den Laser mit Strompulsen betriebt, kann man bei unterschiedlichen Pulsdauern nicht unbedingt von der Erhaltung der Pulsform ausgehen. Dies kann die Auswertung der Messwerte verkomplizieren und ist leicht vermeidbar, wenn man die Pulsdauern alle gleich wählt.

Vorteilhaft ist, den Pulsfolgeabstand so groß zu wählen, dass sich zwischen den einzelnen Pulsen eine möglichst vollständige Abkühlung des Gewebes auf die Basistemperatur einstellt. Alternativ kann auch eine verbleibende Resterwärmung in die Berechnung mit einbezogen werden.

Wie bereits erwähnt ist es ein wichtiger Vorteil der Erfindung, dass die tatsächlich erreichte Zieltemperatur im Gewebe gemessen werden kann, indem die Detektionseinrichtung die Volumenänderungen jeweils bedingt durch die ansteigende und abfallende Flanke des zweiten Heizlaserpulses erfasst und die Messwerte der Recheneinheit zuführt und die Recheneinheit einen Schätzwert für die Temperaturerhöhung während des zweiten Heizlaserpulses ermittelt und anzeigt und/oder protokolliert. Eine solche numerische, objektive Effekteinschätzung für die Laserbehandlung wird als wichtiger Fortschritt gegenüber bisherigen Therapielasern angesehen.

Überdies eröffnet sich mit Leistungslasern der Größenordnungen 5 W bis 100 W auch der Zugang zu thermomechanischen Therapieansätzen, bei denen Zelldisruption durch lokale Verdampfung im Mittelpunkt steht. Auch hierbei ist eine Behandlung und sogar eine gewisse Effektkontrolle mit derselben erfindungsgemäßen Laservorrichtung möglich, wenn lediglich die Software der Recheneinheit erweitert wird.

Vorzugsweise wird in diesem Fall, d.h. bei beabsichtigter thermomechanischer Gewebeschädigung mit einer Zieltemperatur von beispielsweise mehr als 140 °C, die Recheneinheit dazu ausgebildet, ab Beginn des zweiten Heizlaserpulses die Differenz zwischen dem Zeitpunkt des Eintretens einer Volumenänderung an der ansteigenden Flanke des Heizlaserpulses und dem nächstfolgenden Zeitpunkt des Eintretens einer Volumenänderung zu bestimmen und zu protokollieren. Wenn diese gemessene Differenz kleiner als die zweite Pulsdauer ist, dann trifft das die Volumenänderung anzeigenden Messsignal vorzeitig ein und kann somit nicht von der abfallenden Laserflanke herrühren. Vielmehr werden Volumenänderungen erfasst, die auf die Bildung von Mikroblasen im Gewebe zurückgehen. Eine Blasenbildung geht üblich mit Druckamplituden einher, die bis zu zwei Größenordnungen größer als die rein thermisch ausgelösten Druckamplituden sind, wenn die Flanken der Heizlaserpulse nicht zu steil sind. Man kann solche also praktisch nicht übersehen, und der Zeitpunkt ihres Einsetzens markiert den Beginn der Blasenbildung. Wiederum ist die Zeitdifferenz zwischen dem Beginn der Blasenbildung und dem Ende des Heizlaserpulses messbar und kann als ein Maß für die thermomechanische Behandlungsdosis herangezogen werden. Aus der in der Recheneinheit ermittelten Zeitdifferenz zwischen dem Laserpulsanfang und dem Beginn der Blasenbildung ist dieses Maß bei bekannter Pulsdauer unmittelbar ersichtlich.

Die Erfindung wird im Folgenden noch näher erläutert auch anhand von Figuren. Dabei zeigt:

    Fig. 1 eine Skizze des Lasersystems mit Recheneinheit und Detektor für Volumenänderungssignale;

    Fig. 2 experimentelle Daten zu den Flankensteigungen des Laserlichts bei verschiedenen Pulsdauern und Leistungen am Beispiel eines Diodenlasers;

    Fig.3a-3c experimentell gemessene (zeitverschobene) Drucktransienten für Heizlaserpulse vergleichbarer Pulsdauer mit verschiedenen Leistungen.

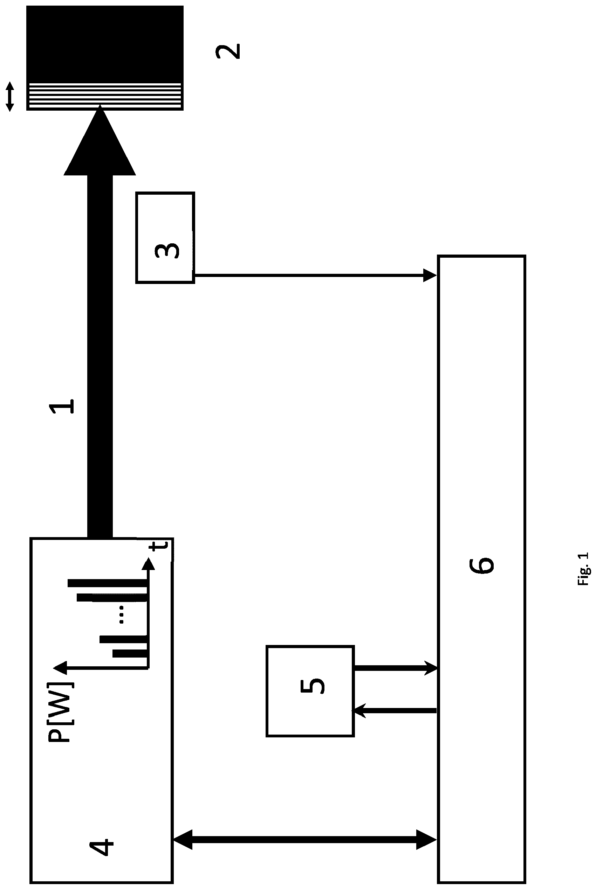

In Fig. 1 ist eine Skizze der erfindungsgemäßen Laservorrichtung in einer Ausgestaltung zur Therapie der Netzhaut zu sehen. In dieser Skizze werden die Mittel zur Lichtführung, der Applikator sowie der Auslöser für den Applikator nicht dargestellt.

Eine Laserquelle (4) emittiert Heizlaserpulse (1) auf Gewebe (2). Eine Detektionseinrichtung registriert die Volumenänderung des Gewebes und erzeugt elektrische Signale, die einer Recheneinheit (6) zugeführt werden. Die Recheneinheit (6) kann ein programmierbarer Mikroprozessor oder Personal Computer sein. Sie verarbeitet die von der Detektionseinrichtung (3) eintreffenden Rohdaten - und speichert diese bei Bedarf nichtflüchtig ab - und bestimmt auf der Basis der Verarbeitungsergebnisse Steuerbefehle an den Laser (4). Eine Steuereinrichtung für den Laser (4) ist nicht separat dargestellt, sondern vielmehr üblich in den Laser (4) in baulicher Einheit integriert. Wenn, wie hier als bevorzugt angenommen, die Betriebsstromstärke des Lasers (4) zur Steuerung der Laserleistung variiert wird, dann kann die Steuereinrichtung als eine einfache analoge Schaltung ausgeführt sein, die unmittelbar aus der Recheneinheit (6) stammenden Spannungswerte in Ströme überführt. Alternativ kann die Steuereinrichtung auch in der Recheneinheit (6) integriert sein. Die Steuereinrichtung kann auch dazu ausgebildet sein, Metabefehle der Recheneinheit (6) wie z.B. Werte für Pulsdauer und Pulsleistung digital entgegenzunehmen und diese in analoge Signale zur Kontrolle des Lasers (4) zu übersetzen.

Der Laser (4) weist erfindungsgemäß eine Emissionsleistung der Größenordnungen 5 W bis 100 W auf. Hier exemplarisch im Laser (4) integriert und nicht eigens dargestellt sind bevorzugt Mittel zur zeitlichen Erfassung der emittierten Laserleistung wenigstens eines Anteils des Laserlichts (1) vorgesehen. Die detektierten Messwerte der Laserleistung werden der Recheneinheit (6) zugeführt, wobei die Recheneinheit (6) für die Laserpulse die Steigungen der Leistungsflanken ermittelt. Eine solche Strahlkontrolle ist gängiger Stand der Technik. Bei sehr stabilen Laserlichtquellen kommt auch das Erstellen einer Tabelle mit Steigungen für Laserpulse verschiedener Pulsdauern und Leistungen anstelle der fortlaufend wiederholten Messung in Betracht.

Eine Bedieneinrichtung (5) vorgesehen zur Nutzereingabe von Bestrahlungsparametern in die Recheneinheit (6) ist vorzugsweise dazu ausgebildet, dem Nutzer auswählbare Intervalle für Bestrahlungsparameter anzuzeigen, wobei die Intervallgrenzen hierarchisch von Nutzereingaben abhängen. Dies bedeutet, dass die Bedieneinrichtung (5) in der Lage sein kann, bestimmte Sicherheitsregeln einzuhalten und diesen Regeln widersprechende Eingaben zu verweigern. Bei der Verwendung eines Leistungslasers mit hohem Schadenspotenzial vor allem bei der Netzhaut-Therapie können so irrtümliche Parametereinstellungen durch den Nutzer frühzeitig abgefangen werden. Beispiels- und vorzugsweise kann die Bedieneinrichtung (5) dazu ausgebildet sein, die Leistung des ersten Heizlaserpulses basierend auf der Nutzereingabe der Pulsdauer so auszuwählen, dass der erste Heizlaserpuls keine dauerhafte Gewebebeschädigung herbeiführt. Weiterhin vorteilhaft ist es, wenn die Bedieneinrichtung (5) dazu ausgebildet ist, als Nutzereingaben für den zeitlichen Pulsabstand zwischen dem ersten und dem wenigstens zweiten Heizlaserpuls nur Werte zuzulassen, die größer sind als eine wenigstens aus der Nutzereingabe für die erste Pulsdauer vorbestimmte thermische Relaxationszeit des Gewebes. Mit hierarchischer Abhängigkeit ist hier gemeint, dass einige Auswahloptionen des Nutzers vorrangig umgesetzt werden, während andere Auswahloptionen als nachrangig gelten und unter Anwendung vorbestimmter - z.B. programmierter - Sicherheitsregeln sachgerecht eingeschränkt werden in Abhängigkeit von den vorrangigen Nutzervorgaben.

Das Bedienelement (5) kann insofern einen eigenen Prozessor zur Datenverarbeitung und einen Datenspeicher zur nicht-flüchtigen Datenspeicherung umfassen.

Das Bedienelement (5) kann so ausgebildet sein, dass als Nutzereingaben wenigstens die Zieltemperatur und die Laserpulsdauer - beispielsweise vorrangig - sowie optional und nachrangig der zeitliche Laserpulsabstand und die erste Leistung des ersten Heizlaserpulses vorgesehen sind. Weiterhin kann der Prozessor des Bedienelementes (5) einen Verschlüsselungsalgorithmus ausführen, um von der Recheneinheit (6) übermittelte Daten zusammen mit den Nutzereingaben zu verschlüsseln und dadurch unseparierbar und unveränderlich zu sichern. Derart gesicherte Daten sind nachträglich nur noch zusammen lesbar und können als authentifizierbares Behandlungsprotokoll (auch mit einem Zeitstempel) dienen.

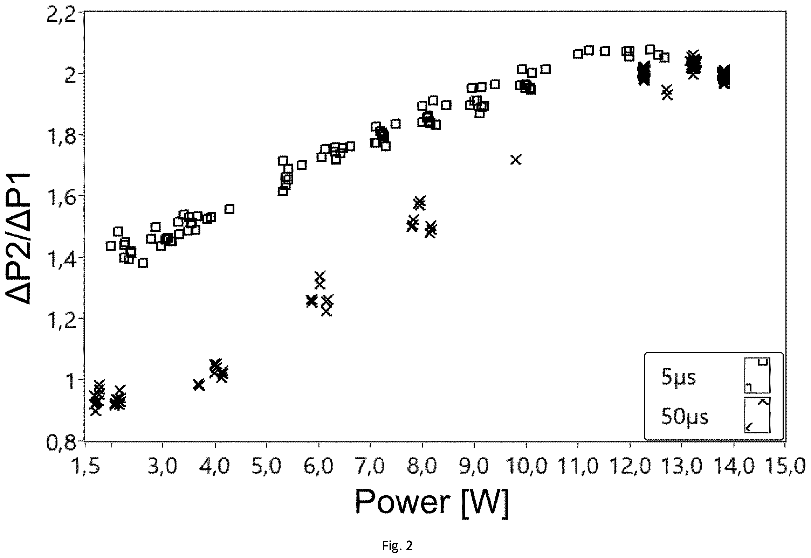

Die Variation des Verhältnisses der Flankensteigungen, $\Delta P_2/\Delta P_1$, der Heizlaserpulse ist bei Variation der Pulsdauer und der Laserleistung experimentell untersucht worden. Fig. 2 zeigt Messwerte für Pulsdauern von 5 µs und 50 µs bei Leistungen bis zu 14 W lediglich zur exemplarischen Illustration. Es zeigt sich, dass sich die Verhältnisse der Flankensteigungen der Pulse - wie auch die Flankensteigungen selbst - mit der applizierten Leistung durch veränderte Anregungsbedingungen (u.a. Pumpstrom) des Lasers ändern. Es wird deutlich, dass die pulsweise Messung der Flankensteigungen für die hier beschriebene Auswertung zweckmäßig ist, wenn man keine umständliche Kalibrierung der Laserlichtquelle durchführen will. Überdies sind Alterungseffekte des Lasers möglich, so dass eine einmalige Kalibrierung ab Werk nicht unbedingt immer ausreicht.

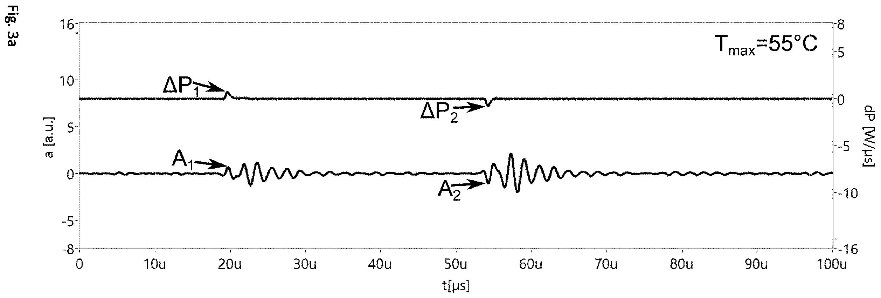

Die Fig. 3a-3c zeigen drei Beispiele für gemessene Drucktransienten bei unterschiedlichen Laserleistungen. Die Drucktransienten sind jeweils die unteren Kurven, die Ableitungen des Heizlaserpulses die oberen. Sie sind zwecks Darstellbarkeit des Vergleichs in allen Plots so normiert worden, dass das erste Maximum der ersten Drucktransienten der maximalen Steigung $\Delta P1$ der ansteigenden Flanke des ersten Heizlaserpulses dem Zahlenwert nach entspricht. So kann leicht erkannt werden, wie hoch der Beitrag einer Temperaturerhöhung zur Drucktransiente am Ende des Heizlaserpulses ist. Die akustische Laufzeit von der Probe zum Sensor wurde zur besseren Darstellung entfernt, d.h. die Drucktransienten wurden um die Laufzeit verschoben.

Fig. 3a zeigt Drucktransienten bei einer Bestrahlung mit 614mJ/cm$^2$: Der eingestrahlte Puls von 35 µs Pulsdauer weist zwei Flankensteigungen $\Delta P1$ und $\Delta P2$ auf, die je eine Drucktransiente A1 und A2 auslösen. Die Drucktransienten sind hier auf derselben Zeitachse wie die Heizlaserpulse dargestellt, also verschoben um die konstante Signallaufzeit von der Netzhaut zum Ultraschallwandler, zur besseren Zuordnung. Den Drucktransienten werden Energiemaßzahlen dD1 und dD2 zugeordnet, die hier beispielsweise aus den Maximalamplituden der Signale A1 und A2 bestimmt werden. Hieraus wird das Verhältnis $\alpha$ berechnet und es ergibt sich $\overline{T_{Helz}}$ als Temperatur am Ende des Pulses zu 55°C. Ein solcher Temperaturanstieg für kurze Zeit schädigt lebendes Netzhautgewebe in der Regel nicht. Die gezeigten Kurven repräsentieren ein Beispiel für den ersten Heizlaserpuls des erfindungsgemäßen Verfahrens.

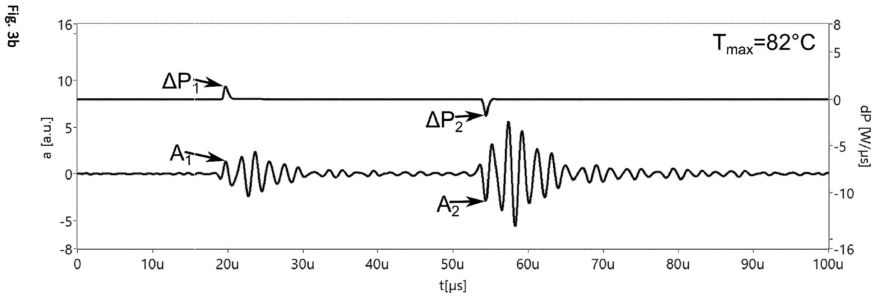

Fig. 3b zeigt Drucktransienten bei einer Bestrahlung mit 1245mJ/cm$^2$: Beim Applizieren eines Pulses der doppelten Leistung im Vergleich zu Fig. 3a zweigen sich dieselben Signale mit nunmehr vergrößerten Werten. Die Laserpulsflanken steigen schneller an, und die Amplituden der Drucktransienten sind deutlich erhöht. Mit denselben Rechnungen wie für Fig. 3a ergibt sich eine Temperatur von 82° C am Pulsende, die bereits im üblichen Bereich der Zieltemperatur einer Photokoagulation liegt. Fig. 3b repräsentiert somit einen möglichen zweiten Heizlaserpuls der Erfindung, der direkt zum therapeutischen Effekt führt.

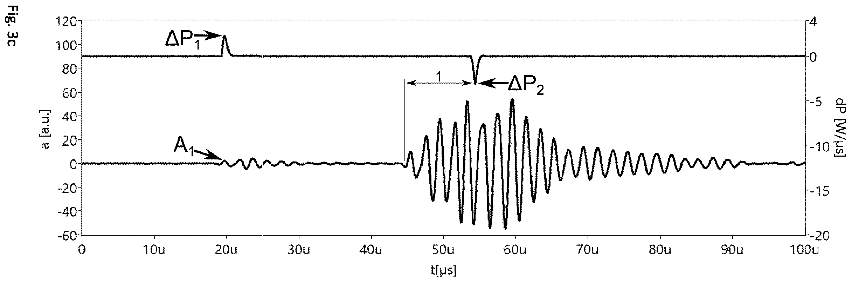

Die Fig. 3c zeigt Drucktransienten bei einer Bestrahlung mit 1488mJ/cm$^2$: Diese Bestrahlung führt zur Blasenbildung im Gewebe. Die zweite Drucktransiente setzt bereits ein, bevor die Laser-Leistung abfällt. Sie beruht somit nicht auf der thermoelastischen Relaxation bei Abkühlung des Gewebes, sondern auf Materialverdampfung. Der Doppelpfeil 1 zeigt die zeitliche Differenz des Einsetzens des akustischen Signals und dem Ende der Bestrahlung. Man beachte die geänderten Skalen gegenüber Fig. 3a und Fig. 3b. Die um den Faktor 10 erhöhte Amplitude ist ebenfalls ein Indiz für Mikroblasenbildung und bestätigt die Annahme. Da die Leistung der Bestrahlung sowie die Zeit zur Mikroblasenbildung bekannt ist, lässt sich bestimmen, mit welcher Bestrahlungsstärke die Blasenbildungstemperatur ab ca. 140°C erreicht werden kann. Ebenso kann die Dauer der thermomechanischen Disruption bis zum Ende des Heizlaserpulses als ein Dosismaß benutzt und ggf. für weitere Laserpulse - durch Einrichten der Leistung - angepasst und optimiert werden.

Es soll hier noch angemerkt sein, dass die Kurven der Fig. 3a-3c nicht am selben Laserspot aufgezeichnet worden sind. Deshalb unterscheiden sich die Absorptionskoeffizienten des Gewebes zwischen den Experimenten.

**Patentansprüche**

1. Lasertherapievorrichtung zur Therapie eines lebenden Gewebes (2) aufweisend eine gepulst betriebene Laserlichtquelle (4) emittierend Laserlicht (1) mit einer Emissionsleistung der Größenordnungen 10 W bis 100 W, eine Einrichtung zur Führung des Laserlichts (1) in einen Applikator, eine Auslöseeinrichtung zum Auslösen der Gewebebestrahlung mit dem Applikator, eine Detektionseinrichtung (3) zur Messung der durch Laserabsorption im Gewebe (2) ausgelösten zeitabhängigen Volumenänderungen, eine Recheneinheit (6) zur Bewertung der detektierten Volumenänderungen und zur Ausgabe von Steuerbefehlen an eine Steuereinrichtung zur Steuerung der in das Gewebe (2) eingestrahlten Leistung des Laserlichts (1), wobei

   a. jedes Auslösen der Gewebebestrahlung die Steuereinrichtung veranlasst, einen ersten Laserpuls mit einer ersten Leistung und einer ersten Pulsdauer und wenigstens einen zweiten Laserpuls mit einer zweiten Leistung und einer zweiten Pulsdauer in einem vorbestimmten zeitlichen Pulsabstand auf das Gewebe (2) zu applizieren, wobei
   b. die Detektionseinrichtung (3) die durch die ansteigende und die abfallende Leistungsflanke des ersten Laserpulses bedingten Volumenänderungen optisch oder akustisch erfasst und die Messwerte der Recheneinheit (6) zuführt;
   c. die Recheneinheit (6) basierend auf den Messwerten der Volumenänderung unter Berücksichtigung wenigstens der vorbestimmten Steigungen der Leistungsflanken des ersten Laserpulses einen Schätzwert für die Temperaturerhöhung des Gewebes (2) während der Einstrahlung des ersten Laserpulses ermittelt und **dadurch gekennzeichnet, dass**
   d. die Recheneinheit (6) aus dem Schätzwert einen Befehl an die Steuereinrichtung erzeugt, der die Steuereinrichtung veranlasst, die zweite Leistung und/oder die zweite Pulsdauer des zweiten Laserpulses so einzurichten, dass die Einstrahlung des zweiten Laserpulses das Gewebe bis auf eine vorbestimmte Zieltemperatur erwärmt.

2. Lasertherapievorrichtung nach Anspruch 1, **dadurch gekennzeichnet, dass** die Steuereinrichtung so eingerichtet ist, dass die Pulsdauern aller bei einer Auslösung applizierten Laserpulse gleich groß sind.

3. Lasertherapievorrichtung nach einem der vorangehenden Ansprüche, **dadurch gekennzeichnet, dass** die Detektionseinrichtung (3) die durch die ansteigende und abfallende Leistungsflanke des zweiten Laserpulses bedingten Volumenänderungen erfasst und die Messwerte der Recheneinheit (6) zuführt und die Recheneinheit (6) einen Schätzwert für die Temperaturerhöhung während der Einstrahlung des zweiten Laserpulses ermittelt und anzeigt und protokolliert.

4. Lasertherapievorrichtung nach einem der vorangehenden Ansprüche, **dadurch gekennzeichnet, dass** Mittel zur zeitlichen Erfassung der emittierten Laserleistung vorgesehen sind, die wenigstens einen Anteil des Laserlichts (1) detektieren und Messwerte der Laserleistung der Recheneinheit (6) zuführen, wobei die Recheneinheit (6) für die Laserpulse die Steigungen der Leistungsflanken ermittelt.

5. Lasertherapievorrichtung nach einem der vorangehenden Ansprüche, **dadurch gekennzeichnet, dass** die Detektionseinrichtung (3) zur Erfassung von thermisch im bestrahlten Gewebe ausgelösten Druckwellen ausgebildet ist und wenigstens einen Ultraschallwandler umfasst, der eine Drucktransiente erfasst und als Messwerte ausgibt.

6. Lasertherapievorrichtung nach einem der Ansprüche 1 bis 4, **dadurch gekennzeichnet, dass** die Detektionseinrichtung (3) zur optischen Erfassung von thermisch im bestrahlten Gewebe ausgelösten Bewegungen von Licht streuenden oder reflektierenden Gewebeschichten ausgebildet ist und ein Interferometer sowie wenigstens einen Photodetektor umfasst, der eine zeitlich veränderliche Lichtintensität erfasst und als Messwerte ausgibt.

7. Lasertherapievorrichtung nach einem der vorangehenden Ansprüche, **dadurch gekennzeichnet, dass** die Recheneinheit dazu ausgebildet ist, ab Beginn des zweiten Laserpulses die Differenz zwischen dem Zeitpunkt des Eintretens einer Volumenänderung an der ansteigenden Flanke des Laserpulses und dem nächstfolgenden Zeitpunkt des Eintretens einer Volumenänderung zu bestimmen und zu protokollieren.

8. Lasertherapievorrichtung nach einem der vorangehenden Ansprüche, **dadurch gekennzeichnet, dass** eine Bedieneinrichtung (5) zur Nutzereingabe von Bestrahlungsparametern in die Recheneinheit (6) dazu ausgebildet ist, dem Nutzer auswählbare Intervalle für Bestrahlungsparameter anzuzeigen, wobei die Intervallgrenzen hierarchisch von Nutzereingaben abhängen.

9. Lasertherapievorrichtung nach Anspruch 9, **dadurch gekennzeichnet, dass** die Bedieneinrichtung (5) dazu ausgebildet ist, die Leistung des ersten Laserpulses basierend auf der Nutzereingabe der Pulsdauer so auszuwählen, dass der erste Laserpuls keine dauerhafte Gewebebeschädigung herbeiführt.

10. Lasertherapievorrichtung nach einem der Ansprüche 9 oder 10, **dadurch gekennzeichnet, dass** die Bedieneinrichtung (5) dazu ausgebildet ist, als Nutzereingaben für den zeitlichen Pulsabstand zwischen dem ersten und dem wenigstens zweiten Laserpuls nur Werte zuzulassen, die größer sind als eine wenigstens aus der Nutzereingabe für die erste Pulsdauer vorbestimmte thermische Relaxationszeit des Gewebes.

**Claims**

1. Laser therapy device for the therapy of a living tissue (2) comprising a pulsed operated laser light source (4) emitting laser light (1) with an emission power of the order of magnitude of 10 W to 100 W, a device for guiding the laser light (1) into an applicator, a trigger device for triggering tissue irradiation with the applicator, a detection device (3) for measuring the time-dependent volume changes triggered by laser absorption in the tissue (2), a computing unit (6) for evaluating the detected volume changes and for issuing control commands to a control device for controlling the power of the laser light (1) irradiated into the tissue (2), wherein

   a. each triggering of the tissue irradiation causes the control device to apply a first laser pulse with a first power and a first pulse duration, and at least a second laser pulse with a second power and a second pulse duration at a predetermined time interval to the tissue (2), wherein
   b. the detection device (3) optically or acoustically records the volume changes caused by the rising and falling power flank of the laser pulse, and supplies the measurement to the computing unit (6);
   c. the computing unit (6), on the basis of the measurements of the volume change, taking into account at least the predetermined rises of the power edges of the first laser pulse, determines an estimated value for the temperature increase of the tissue (2) during the first laser pulse irradiation and **characterised in that**
   d. from the estimated value, the computing unit (6) generates a command to the control device, which causes the control device to set the second power and/or the second pulse duration of the second laser pulse so the irradiation of the second laser pulse heats the tissue to a predetermined target temperature.

2. Laser therapy device according to claim 1, **characterised in that** the control device is configured in such a way that the pulse durations of all laser pulses applied at the time of triggering are the same.

3. Laser therapy device according to any one of the preceding claims, **characterised in that** the detection device (3) records the volume changes caused by the rising and falling power edge of the laser pulse, and supplies the measurement to the computing unit (6) and the computing unit (6) determines, displays and logs an estimated value for the temperature increase during irradiation by the second laser pulse.

4. Laser therapy device according to any one of the preceding claims, **characterised in that** means are provided for the temporal recording of the emitted laser power which detects at least a portion of the laser light (1) and transmits measurements of the laser power to the computing unit (6), wherein for the laser pulses the computing unit (6) determines the rises of the power edges.

5. Laser therapy device according to any one of the preceding claims, **characterised in that** detection device (3) is designed to record pressure waves thermally triggered in the irradiated tissue and comprises at least one ultrasonic transducer that records a pressure transient and emits it as measuring values.

6. Laser therapy device according to any one of claims 1 to 4, **characterised in that** the detection device (3) can be designed, for example, for the optical detection of thermally triggered movements of light-scattering or reflecting tissue layers in the irradiated tissue and comprises an interferometer as well as at least one photodetector which measures a temporally changeable light intensity and emits this as a measuring values.

7. Laser therapy device according to any one of the preceding claims, **characterised in that** as of the start of the second laser pulse, the computing unit is designed to determine and log the difference between the time of occurrence of a change in volume at the rising edge of the laser pulse and the next time of occurrence of a change in volume.

**8.** Laser therapy device according to any of the preceding claims, **characterised in that** an operating device (5) for the user input of irradiation parameters into the computing unit (6) is designed to display to the user selectable intervals for irradiation parameters, wherein the interval limits depend hierarchically on user inputs.

**9.** Laser therapy device according to claim 9, **characterised in that** the control device (5) is designed to select the power of the first laser pulse based on the user input of the pulse duration in such a way that the first laser pulse does not cause lasting tissue damage.

**10.** Laser therapy device according to any one of claims 9 or 10, **characterised in that** the operating device (5) is designed to only allow as user inputs for the temporal pulse interval between the first and at least the second laser pulse, values that are greater than a thermal relaxation time of the tissue predetermined at least from the user input for the first pulse duration.

**Revendications**

**1.** Dispositif de thérapie au laser destiné à la thérapie d'un tissu vivant (2) comportant une source de lumière laser à fonctionnement pulsé (4) émettant de la lumière laser (1) avec une puissance d'émission ayant des ordres de grandeur de 10 W à 100 W, un système pour le guidage de la lumière laser (1) dans un applicateur, un système de déclenchement pour déclencher l'irradiation du tissu avec l'applicateur, un système de détection (3) pour mesurer les variations de volume en fonction du temps déclenchées par l'absorption laser dans le tissu (2), une unité de calcul (6) pour évaluer les variations de volume détectées et pour émettre des ordres de commande à un système de commande pour commander la puissance irradiée dans le tissu (2) de la lumière laser (1), sachant que

a. chaque déclenchement de l'irradiation du tissu incite le système de commande à appliquer une première impulsion laser avec une première puissance et une première durée d'impulsion et au moins une deuxième impulsion laser avec une deuxième puissance et une deuxième durée d'impulsion dans un intervalle de temps d'impulsion prédéterminé sur le tissu (2), sachant que
b. le système de détection (3) saisit de façon optique ou acoustique les variations de volume conditionnées par les flancs de puissance ascendants ou descendants de la première impulsion laser et acheminer les valeurs de mesure à l'unité de calcul (6),

c. l'unité de calcul (6) détermine une valeur d'estimation pour la hausse de température du tissu (2) pendant l'irradiation de la première impulsion laser en se basant sur les valeurs de mesure de la variation de volume en tenant compte au moins des inclinaisons prédéterminées des flancs de puissance de la première impulsion laser et **caractérisé en ce que**
d. l'unité de calcul (6) produit à partir de la valeur d'estimation un ordre destiné au système de commande, qui incite le système de commande à instaurer la deuxième puissance et/ou la deuxième durée d'impulsion de la deuxième impulsion laser de telle manière que l'irradiation de la deuxième impulsion laser réchauffe le tissu jusqu'à une température visée prédéterminée.

**2.** Dispositif de thérapie au laser selon la revendication 1, **caractérisé en ce que** le système de commande est agencé de telle manière que les durées d'impulsion de toutes les impulsions laser appliquées lors d'un déclenchement sont de la même importance.

**3.** Dispositif de thérapie au laser selon l'une quelconque des revendications précédentes, **caractérisé en ce que** le système de détection (3) saisit les variations de volumes conditionnées par les flancs de puissance ascendants et descendants de la deuxième impulsion laser et achemine les valeurs de mesure à l'unité de calcul (6) et l'unité de calcul (6) détermine, affiche et enregistre une valeur d'estimation pour la hausse de température pendant l'irradiation de la deuxième impulsion laser.

**4.** Dispositif de thérapie au laser selon l'une quelconque des revendications précédentes, **caractérisé en ce que** des moyens de saisie temporelle de la puissance de laser émise sont prévus, qui détectent au moins une partie de la lumière laser (1) et achemine les valeurs de mesure de la puissance de laser à l'unité de calcul (6), sachant que l'unité de calcul (6) pour les impulsions laser détermine les inclinaisons des flancs de puissance.

**5.** Dispositif de thérapie au laser selon l'une quelconque des revendications précédentes, **caractérisé en ce que** le système de détection (3) est constitué pour la saisie d'ondes de pression déclenchées thermiquement dans le tissu irradié et comprend au moins un transducteur à ultrasons, qui saisit une transitoire de pression et la restitue sous la forme de valeurs de mesure.

**6.** Dispositif de thérapie au laser selon l'une quelconque des revendications 1 à 4, **caractérisé en ce que** le système de détection (3) est constitué pour la saisie optique de mouvements déclenchés thermiquement dans le tissu irradié par des couches de tissu dispersant ou réfléchissant la lumière et comprend un interféromètre et ainsi qu'au moins un photodétecteur, qui saisit une intensité lumineuse variable dans le temps et la restitue sous la forme de valeurs de mesure.

**7.** Dispositif de thérapie au laser selon l'une quelconque des revendications précédentes, **caractérisé en ce que** l'unité de calcul est constituée pour déterminer et enregistrer à compter du début de la deuxième impulsion laser, la différence entre le moment de l'apparition d'une variation de volume sur les flancs ascendants de l'impulsion laser et le moment immédiatement suivant de l'apparition d'une variation de volume.

**8.** Dispositif de thérapie au laser selon l'une quelconque des revendications précédentes, **caractérisé en ce qu'**un système d'actionnement (5) pour l'entrée d'utilisateur de paramètres d'irradiation dans l'unité de calcul (6) est constitué pour afficher à l'utilisateur les intervalles sélectionnables pour les paramètres d'irradiation, sachant que les limites d'intervalle dépendent de façon hiérarchique des entrées d'utilisateur.

**9.** Dispositif de thérapie au laser selon la revendication 9, **caractérisé en ce que** le système d'actionnement (5) est constitué pour sélectionner la puissance de la première impulsion laser en se basant sur l'entrée d'utilisateur de la durée d'impulsion de telle manière que la première impulsion laser n'entraîne aucune lésion tissulaire durable.

**10.** Dispositif de thérapie au laser selon l'une quelconque des revendications 9 ou 10, **caractérisé en ce que** le système d'actionnement (5) est constitué pour ne permettre en tant qu'entrées d'utilisateur pour l'intervalle d'impulsion temporel entre la première et au moins la deuxième impulsion laser que des valeurs qui sont plus grandes qu'un temps de relaxation thermique du tissu prédéterminé au moins à partir de l'entrée d'utilisateur pour la première durée d'impulsion.

Fig. 1

EP 4 288 007 B1

Fig. 2

Fig. 3a

Fig. 3b

Fig. 3c

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2003032949 A1
- US 2010292763 A1
- US 2013102894 A1
- US 2015011983 A1
- DE 102009016184 A1

**In der Beschreibung aufgeführte Nicht-Patentliteratur**

- **XIAOHUA FENG et al.** Selftemperature regulation of photothermal therapy by laser-shared photoacoustic feedback. *Optics Letters,* 01. Oktober 2015, vol. 40 (19), 4492-4495
- **FEI GAO et al.** Single laser pulse generates dual photoacoustic signals for differential contrast photoacoustic imaging. *Scientific Reports,* 04. April 2017, vol. 7, 626
- **BRINKMANN ; BIRNGRUBER.** Selektive Retina-Therapie. *Z. Med. Phys,* 2007, vol. 17, 6-22
- **KANDULLA et al.** *Journal of Biomedical Optics,* 2006, vol. 11 (4